# EUROPEAN PATENT SPECIFICATION

(11) **EP 4 591 893 B1**
(45) Date of publication and mention of the grant of the patent: **21.10.2026**
(21) Application number: 24855110.3
(22) Date of filing: 13.12.2024
(51) Int. Cl.: A61L 27/18, A61L 27/50, A61L 27/54

(54) **BIOMATERIAL COMPOSITION FOR TISSUE REPAIR COMPRISING DNA FRAGMENT MIXTURE AND POLYHYDRIC ALCOHOL**
BIOMATERIALZUSAMMENSETZUNG ZUR GEWEBEREPARATUR MIT DNA-FRAGMENTMISCHUNG UND POLYHYDRISCHEM ALKOHOL
COMPOSITION DE BIOMATÉRIAU POUR RÉPARATION TISSULAIRE COMPRENANT UN MÉLANGE DE FRAGMENTS D'ADN ET UN ALCOOL POLYHYDRIQUE

(30) Priority: 15.12.2023 KR 20230183306
(43) Date of publication of application: 30.07.2025
(73) Proprietor: PharmaResearch Co., Ltd., Gangneung-si, Gangwon-do 25452 (KR)
(72) Inventor: PARK, Sangun, Seongnam-si, Gyeonggi-do 13453 (KR); SEO, Jeongwoo, Seongnam-si, Gyeonggi-do 13453 (KR); CHUN, Soyoung, Seongnam-si, Gyeonggi-do 13453 (KR); JEONG, Da-won, Seongnam-si, Gyeonggi-do 13453 (KR)
(74) Representative: Cabinet Beau de Loménie
(86) International application number: PCT/KR2024/096943
(87) International publication number: WO 2025/127873

(56) References cited:
- KR-A- 20190 102 967
- KR-A- 20200 031 229
- KR-A- 20210 135 529
- KR-B1- 101 710 639
- KR-B1- 101 722 181
- KR-B1- 102 503 635
- US-A1- 2014 057 973

## Description

### [Technical Field]

The present disclosure relates to a composition for tissue repair; a filler composition; and a biomaterial for tissue repair.

### [Background Art]

With the growing interest in anti-aging, there is a growing number of procedures that aim to compensate for deficiencies in the body, comprising joints, cardiovascular, skin, etc. For example, cosmetic procedures aimed at improving wrinkles or other appearance conditions are also one of them. In this regard, a method of injecting highly biocompatible materials has been frequently performed in recent years so as to replace a damaged biological tissue and to add volume in a desired body area.

For example, there are filler compositions having hyaluronic acid as a main component, but some hyaluronic acid fillers are difficult to inject into the skin due to their high viscosity and low elasticity, and even when injected into the skin, they do not retain their injected form (shape) for a long time and have a short shape retention period. To improve this, various compounds are being added (US Patent No. 11,154,481 and KR101710639 B1), but it is rather difficult to improve the problem while minimizing side effects.

### [Disclosure]

### [Technical Problem]

There is still a need to develop improved biomaterials for tissue repair.

### [Technical Solution]

An object of the present disclosure is to provide a composition for tissue repair, the composition comprising a DNA fragment mixture; and a C3 or C4 polyhydric alcohol, wherein the DNA fragment mixture is comprised in an amount of 2% by weight to 5% by weight with respect to the total weight of the composition, and the polyhydric alcohol is comprised in an amount of 0.5% by weight to 4% by weight with respect to the total weight of the composition, and the polyhydric alcohol is any one or more selected from the group consisting of glycerin, propylene glycol, and butylene glycol.

Another object of the present disclosure is to provide a filler composition comprising a DNA fragment mixture; and a C3 or C4 polyhydric alcohol, wherein the DNA fragment mixture is comprised in an amount of 2% by weight to 5% by weight with respect to the total weight of the composition, and the polyhydric alcohol is comprised in an amount of 0.5% by weight to 4% by weight with respect to the total weight of the composition, and the polyhydric alcohol is any one or more selected from the group consisting of glycerin, propylene glycol, and butylene glycol.

Still another object of the present disclosure is to provide a method of repairing a tissue, the method comprising the step of administering the composition for tissue repair or the filler composition into a subject. However, this method is not according to the invention as claimed.

Still another object of the present disclosure is to provide use of a composition in tissue repair, the composition comprising a DNA fragment mixture; and a C3 or C4 polyhydric alcohol, wherein the DNA fragment mixture is comprised in an amount of 2% by weight to 5% by weight with respect to the total weight of the composition, and the polyhydric alcohol is comprised in an amount of 0.5% by weight to 4% by weight with respect to the total weight of the composition. However, this use is not according to the invention as claimed.

### [Advantageous Effects]

A biomaterial for tissue repair of the present disclosure not only has the ability to repair tissue but also has a moisturizing effect, thereby having an effect of not imparting a drying sensation to the tissue.

### [Brief Description of the Drawing]

FIG. 1 shows the results of verifying the effect of increasing the moisture content through animal testing;
FIG. 2 shows the standardized results of an injection force test for prepared Comparative Examples and Examples;
FIGS. 3 to 6 show the results of a phase angle test for the prepared Comparative Examples and Examples;
FIG. 7 shows the results of testing biodegradability for the prepared Comparative Examples and Examples;
FIG. 8 shows the results of comparing the height and width of the injection site after injecting liquid compositions of the prepared Comparative Examples and Examples; and
FIG. 9 shows the results of comparing properties of each composition when preparing the liquid composition.

### [Detailed Description of Preferred Embodiments]

The present disclosure will be described in detail as follows. The scope of the present disclosure is not limited by the specific description described below.

An aspect of the present disclosure provides a composition for tissue repair, the composition comprising a DNA fragment mixture and an alcohol. Specifically, the alcohol is a C3 or C4 polyhydric alcohol and is any one or more selected from the group consisting of glycerin, propylene glycol, and butylene glycol.

In one embodiment, the composition for tissue repair of the present disclosure is characterized by comprising a mixture of DNA fragments and an active ingredient consisting of a C3 or C4 polyhydric alcohol is any one or more selected from the group consisting of glycerin, propylene glycol, and butylene glycol.

As used herein, the term "DNA fragment mixture" comprises DNA corresponding to a biopolymer composed of phosphate, four types of bases, and deoxyribose, and refers to existence of a mixture of nucleic acid fragments having a molecular weight size in a predetermined range while existing as a polymer of nucleotides. The DNA fragment mixture may exist in a mixed form of fragments with a reduced molecular weight, but is not limited thereto, and may be used interchangeably with terms such as 'DNA fragment', 'DNA fraction', 'nucleic acid fragment', and 'nucleic acid fragment mixture'.

In one embodiment, the DNA fragment mixture of the present disclosure may be polynucleotide (PN), polydeoxyribonucleotide (PDRN), or a mixture thereof.

As used herein, the term "polynucleotide" is referred to as "PN", and may mean a DNA or RNA strand, which is a polymer of nucleotides in which nucleotide units (monomers) are linked together in a chain shape by covalent bonds. Further, as used herein, the term "polydeoxyribonucleotide" is referred to as "PDRN", and may be a kind of low molecular weight DNA complex having a specific molecular weight, but is not limited thereto. For example, the polynucleotide may have a relatively long nucleic acid length or large molecular weight, as compared to polydeoxyribonucleotide, and may be used as a raw material for a medical device with a physical support role for cell fixation and lubrication and buffering effects, and the polydeoxyribonucleotide may be used as a pharmaceutical raw material for cell proliferation and tissue regeneration, but is not limited thereto.

In one embodiment, a liquid composition of the DNA fragment mixture of the present disclosure may comprise a buffer, and the above-described buffer solution may be any one or more selected from the group consisting of sodium phosphate monobasic dihydrate, sodium phosphate dibasic dodecahydrate, sodium chloride, magnesium chloride, potassium chloride, and phosphate buffer saline, or N-(2-hydroxyethyl)-piperazine-N'-2-ethanesulfonic acid (HEPES), but is not limited thereto.

The DNA fragment mixture according to any one of the preceding embodiments may be obtained by extracting from the testis or semen of a fish. Specifically, the fish may be a salmonid fish. More specifically, it may be salmon or trout, but is not limited thereto.

The DNA fragment mixture according to any one of the preceding embodiments may have a molecular weight of about 1 kDa to about 100,000 kDa, about 5 kDa to about 50,000 kDa, about 50 kDa to about 10,000 kDa, or about 50 kDa to about 1,500 kDa.

In any one of the preceding embodiments, the DNA fragment mixture of the present disclosure may be a polynucleotide (PN), but is not limited thereto.

The DNA fragment mixture according to any one of the preceding embodiments may be comprised in an amount of 2% by weight to 5% by weight, 2% by weight to 4% by weight, 2% by weight to 3% by weight, 3% by weight to 5% by weight, 3% by weight to 4% by weight, 4% by weight to 5% by weight, with respect to the total weight of the composition for tissue repair.

The C3 or C4 polyhydric alcohol of the present disclosure means an alcohol having 3 or 4 carbon atoms while having two or more hydroxyl groups (-OH).

The polyhydric alcohol according to any one of the preceding embodiments may be comprised in an amount of 0.5% by weight to 4% by weight, 0.5% by weight to 3% by weight, 0.5% by weight to 2% by weight, 0.5% by weight to 1% by weight, 1% by weight to 4% by weight, 1 % by weight to 3% by weight, 1% by weight to 2% by weight, 2% by weight to 4% by weight, 2% by weight to 3% by weight, 3% by weight to 4% by weight, with respect to the total weight of the composition for tissue repair.

The polyhydric alcohol according to the invention is one or more selected from the group consisting of glycerin, propylene glycol, and butylene glycol.

In the present disclosure, it was confirmed that the polyhydric alcohol alone may not be used for tissue repair purposes, whereas a combination of the DNA fragment mixture and C3 or C4 polyhydric alcohol may reduce viscosity, thereby increasing ease of injection.

In addition, a combination of the DNA fragment mixture and polyhydric alcohol such as polyethylene glycol (PEG) and triethylene glycol generates a large amount of foam during the preparation of a liquid composition thereof, which increases the probability of defective products during future production of products, or the viscosity approaches 0, making it impossible to use for tissue repair. In contrast, it was confirmed that the combination of the DNA fragment mixture and C3 or C4 polyhydric alcohol of the present disclosure has a significantly low possibility thereof.

Meanwhile, the composition for tissue repair of the present disclosure may have tissue moisturizing ability.

As used herein, the term "tissue moisturizing ability" means increasing the moisture content of the tissue. The tissue moisturizing ability of the present disclosure may mean that the moisture content of the tissue increases when the composition of the present disclosure is injected into the tissue, as compared to the moisture content when it is not injected into the tissue. Such moisturizing ability may contribute to tissue repair with a high engraftment rate and moisturizing while increasing the moisture content in the tissue and not giving a dry feeling to the surrounding tissue but rather relieving the dry feeling.

The composition for tissue repair according to any one of the preceding embodiments may increase the moisture content of the tissue upon tissue injection.

Examples of the tissues may comprise, but are not limited to, skin.

In one embodiment of the present disclosure, it was confirmed that the composition for tissue repair further comprising C3 or C4 polyhydric alcohol (especially, at a specific content) not only had an excellent tissue repair effect but also increased the moisture content, when injected into tissues, as compared to comprising the DNA fragment mixture alone. This suggests that the composition of the present disclosure, when injected, does not excessively absorb moisture from surrounding tissues, provides moisture for the surrounding tissues, does not cause a dry feeling to the surrounding tissues, but rather alleviates the dry feeling, and has a tissue repair effect with a high engraftment rate and a good moisture content.

In particular, it was confirmed that there is a critical significance for a moisturizing effect when the composition comprises the DNA fragment mixture; and C3 or C4 polyhydric alcohol, wherein the DNA fragment mixture is comprised in an amount of 2% by weight to 5% by weight with respect to the total weight of the composition, and the polyhydric alcohol is comprised in an amount of 0.5 to 4% by weight with respect to the total weight of the composition, and the tissue repair effect is also excellent in the same range. This suggests that in the range of the above-mentioned content combination, the composition does not give a dry feeling to the surrounding tissue, but rather alleviates the dry feeling and has a tissue repair effect with a good moisturizing effect.

The composition for tissue repair according to any one of the preceding embodiments may comprise the DNA fragment mixture and the polyhydric alcohol at a weight ratio of 10:1 to 1:2, but is not limited thereto.

In any one of the preceding embodiments, the composition for tissue repair of the present disclosure may comprise the DNA fragment mixture and the polyhydric alcohol at a weight ratio of about 10:1 to about 1:2, about 10:1 to about 1:1, about 8:1 to about 1:2, about 8:1 to about 1:1, about 6:1 to about 1:2, about 6:1 to about 1:1, about 5:1 to about 1:2, about 5:1 to about 1:1, about 4:1 to about 1:2, or about 4:1 to about 1:1, specifically, at a weight ratio of about 10:1 to about 1:2, about 10:1 to 1:1, about or 6:1 to 1:2.

The term "about" may comprise not only the exact number recited after the term, but also a range that is near or close to that number. Considering the context in which the number is presented, it may be determined whether any number is close to or near the particular number presented. For example, the term "about" may refer to a range from -10% to +10% of a numerical value. For another example, the term "about" may refer to a range from -5% to +5% of a given numerical value. For still another example, the term may refer to a range comprising ±0.5, ±0.4, ±0.3, ±0.2, ±0.1, etc., but is not limited thereto.

In the present disclosure, even though the term "about" is omitted before a number, it is obvious that the present disclosure comprises the range in which the term is not omitted.

Meanwhile, as used herein, the term "for tissue repair" means that it may be used for replacement, repair, and/or reconstruction of human tissues and organs, such as blood vessels, heart, septum, fascia, and/or skin, etc.

The composition for tissue repair of the present disclosure may be used as a biomaterial for tissue repair, and the biomaterial for tissue repair means a bioderived material used for replacement, repair, and reconstruction of human tissues and organs such as blood vessels, heart, septum, fascia, skin, etc., and is not limited to a cosmetic filler, and may mean a biomaterial for tissue repair specified by the Ministry of Food and Drug Safety.

The composition for tissue repair of the present disclosure may be a liquid composition or an injectable composition, but is not limited thereto.

As used herein, the term "injectable" means a material having the properties required to administer the composition to a subject using an injectable device having a needle.

As used herein, the term "administrating" means introducing the composition of the present disclosure to a subject by any appropriate method, and the administration may be performed through various routes, such as application, subcutaneous, dermal, blood vessel, biological membrane, tissue fiber, synovial fluid, etc., as long as it may reach a target tissue. The dosage form may be for, but is not limited to, topical application, subcutaneous injection, dermal injection, intravascular injection, intramuscular injection, intraarticular injection, intratendinous injection, and intraligamentous injection. The desirable dosage of the composition of the present disclosure may vary depending on a subject's conditions.

The composition for tissue repair according to the present disclosure may exhibit a suitable range of viscosity for injection into a target tissue.

Further, as used herein, the term 'viscosity' means a property of a fluid, i.e., a flow with viscosity, which is a resistance to flow. This viscosity may be expressed as viscosity, in particular, complex viscosity (η*, Pa·s).

In any one of the preceding embodiments, the composition for tissue repair of the present disclosure may exhibit complex viscosity of 1 Pa·s to 2,000 Pa·s, specifically, 5 Pa·s to 2,000 Pa·s, 8 Pa·s to 2,000 Pa·s, 1 Pa·s to 1,500 Pa·s, 5 Pa·s to 1,500 Pa·s, 8 Pa·s to 1,500 Pa·s, 1 Pa·s to 1,300 Pa·s, 5 Pa·s to 1,300 Pa·s, or 8 Pa·s to 1,300 Pa·s.

The viscosity range of the composition for tissue repair used as a general biomaterial for tissue repair is about 1 Pa·s or more to about 8,000 Pa·s or less. When the viscosity is in the above range, the composition may be naturally engrafted into the tissue in the body after administration. In general, when the viscosity range of the composition for tissue repair is about 1 Pa·s or less, although it is engrafted into the body, its ability to form its own shape is low, and therefore, the composition may not function as a biomaterial for tissue repair to create volume, and there may be difficulties in controlling the injection amount, such as a large amount of injection solution being injected even with a small amount of force. In addition, when the viscosity range is high, such as about 8,000 Pa·s or more, it is difficult to extrude a syringe when injecting the composition into the body through the syringe, and excessive force may be required during injection, making it difficult to inject an accurate amount, and it is difficult to precisely control the injection amount, which may cause side effects.

The composition for tissue repair of the present disclosure may be injected at an injection force of 0 or more; and an extrusion force of about 60 N, about 55 N, about 50 N, about 45 N, about 40 N, about 35 N, about 30 N, or about 25 N or less when administered at a rate of 50 mm/min. For example, the above-mentioned injection force may be by injection through a 33-gauge needle, but is not limited thereto. Specifically, it may be appropriate that the injection force is set at 40 N or less, based on the guideline on approval and review for fillers for plastic surgery.

The composition for tissue repair of the present disclosure may further comprise one or more compounds selected from the group consisting of anesthetics, vitamins, amino acids, metals, antioxidants, and mineral salts, but is not limited thereto.

The composition for tissue repair of the present disclosure may further comprise any suitable excipient commonly used in the art, and such excipients may be, for example, preservatives, wetting agents, dispersing agents, suspending agents, buffering agents, stabilizers, or isotonic agents, but are not limited thereto.

Another aspect of the present disclosure provides a biomaterial for tissue repair, the biomaterial comprising the composition for tissue repair of the present disclosure.

The composition for tissue repair and the biomaterial for tissue repair are as described in other aspects.

Still another aspect of the present disclosure provides a filler composition comprising a DNA fragment mixture; and a polyhydric alcohol, more specifically, a C3 or C4 polyhydric alcohol selected from glycerin, propylene glycol, and butylene glycol.

Further, the DNA fragment mixture may be comprised in an amount of 2% by weight to 5% by weight with respect to the total weight of the composition, and the polyhydric alcohol may be comprised in an amount of 0.5% by weight to 4% by weight with respect to the total weight of the composition.

As used herein, the term "filler", which is a material for tissue repair, refers to a medical appliance that is injected into the skin to restore volume, etc., and has an action principle of maintaining skin volume through physical repair.

The DNA fragment mixture, the polyhydric alcohol, etc. are as described in other aspects, and the filler composition may be a specific example of the composition for tissue repair, and the descriptions regarding the composition for tissue repair of the present disclosure (DNA fragment mixture, polyhydric alcohol, content thereof, weight ratio, tissue moisturizing ability, liquid composition, injectable composition, administration, appropriate range of injectable viscosity, etc.) may all be applied to the filler composition.

The filler composition of the present disclosure may further comprise one or more compounds selected from the group consisting of anesthetics, vitamins, amino acids, metals, antioxidants, and mineral salts, but is not limited thereto.

The filler composition of the present disclosure may further comprise any suitable excipient commonly used in the art, and such excipients may be, for example, preservatives, wetting agents, dispersing agents, suspending agents, buffering agents, stabilizers, or isotonic agents, but are not limited thereto.

Still another aspect of the present disclosure provides a method of repairing a tissue, the method comprising the step of administering the composition for tissue repair or filler composition of the present disclosure into a subject. This method of treatment is not according to the invention as claimed.

The administration, the tissue repair, the composition for tissue repair, the filler composition, etc. are as described in other aspects.

As used herein, the term "subject" means all animals comprising humans, such as rats, mice, livestock, etc., that require or are likely to require tissue repair. Specifically, the subject may be a mammal, comprising humans.

Still another aspect of the present disclosure provides use of a composition in tissue repair, the composition comprising a DNA fragment mixture; and a C3 or C4 polyhydric alcohol, wherein the DNA fragment mixture is comprised in an amount of 2% by weight to 5% by weight with respect to the total weight of the composition, and the polyhydric alcohol is comprised in an amount of 0.5% by weight to 4% by weight with respect to the total weight of the composition.

The DNA fragment mixture, the polyhydric alcohol, and the tissue repair, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Preparation Example: Preparation of Biomaterial for Tissue Repair comprising DNA fragment mixture and Polyhydric alcohol

A DNA fragment mixture was added to buffer and dissolved at a high temperature of 60°C to 80°C using a heat stirrer to prepare a DNA fragment mixture solution. Polyhydric alcohol was added to the DNA fragment mixture solution prepared in the heat stirrer at 60°C to 80°C, and mixed, and then the temperature of the mixed solution was lowered to room temperature to prepare a liquid composition.

At this time, polynucleotide (PN; manufacturer: PharmaResearch Co., Ltd.) was used as a representative example of the DNA fragment mixture, and the name of sample and concentration is as shown in Tables 1 and 2 below.

**[Table 1]**

| Name of sample | DNA fragment mixture (wt%) | Kind and content of polyhydric alcohol (wt%) |
|---|---|---|
| Example 1 | 2 | Glycerin; 2 |
| Example 2 | 2 | Propylene glycol; 2 |
| Example 3 | 2 | Butylene glycol; 2 |
| Example 4 | 2 | PEG400; 2 |
| Example 5 | 2 | PEG4000; 2 |
| Example 6 | 2 | PEG6000; 2 |
| Example 7 | 2 | Triethylene glycol; 2 |

| | | |
|---|---|---|
| Examples 4-7 are comparative examples. | | |

**[Table 2]**

| Name of sample | DNA fragment mixture (wt%) | Content of glycerin (wt%) |
|---|---|---|
| Comparative Example 1 | 2 | 0 |
| Example 8 | 2 | 0.5 |
| Example 9 | 2 | 1.0 |
| Example 10* | 2 | 2.0 |
| Example 11 | 2 | 3.0 |
| Example 12 | 2 | 4.0 |
| Example 13 | 2 | 8.0 |
| Comparative Example 2 | 3 | 0 |
| Example 14 | 3 | 0.5 |
| Example 15 | 3 | 1.0 |
| Example 16 | 3 | 2.0 |
| Example 17 | 3 | 3.0 |
| Example 18 | 3 | 4.0 |
| Example 19 | 3 | 8.0 |
| Comparative Example 3 | 4 | 0 |
| Example 20 | 4 | 0.5 |
| Example 21 | 4 | 1.0 |
| Example 22 | 4 | 2.0 |
| Example 23 | 4 | 3.0 |
| Example 24 | 4 | 4.0 |
| Example 25 | 4 | 8.0 |
| Comparative Example 4 | 5 | 0 |
| Example 26 | 5 | 0.5 |
| Example 27 | 5 | 1.0 |
| Example 28 | 5 | 2.0 |
| Example 29 | 5 | 3.0 |
| Example 30 | 5 | 4.0 |
| Example 31 | 5 | 8.0 |
| Comparative Example 5 | 7 | 0 |
| Example 32 | 7 | 4.0 |
| Example 33 | 7 | 8.0 |
| Comparative Example 6 | 0 | 2.0 |

| | | |
|---|---|---|
| * Example 10 has the same composition and content as Example 1. Examples 13, 19, 25 and 31-33 are comparative examples. | | |

### Experimental Example 1: Verification of Moisturizing Effect through Animal Testing

A skin moisture loss model was prepared as a representative example of tissue using mice. At this time, the model was prepared by referring to the already known method (Park, No-June, et al. "Compound K improves skin barrier function by increasing SPINK5 expression." Journal of Ginseng Research 44.6 (2020): 799-807).

Into the body of the prepared animal model, the liquid composition prepared in Preparation Example was injected, and then the moisture content of the mouse skin was measured under conditions of 25±5°C and 50% ±5% RH using a hydration measurement device (Corneometer^{®} CM 825 (Courage + Khazaka electronic GmbH, Germany).

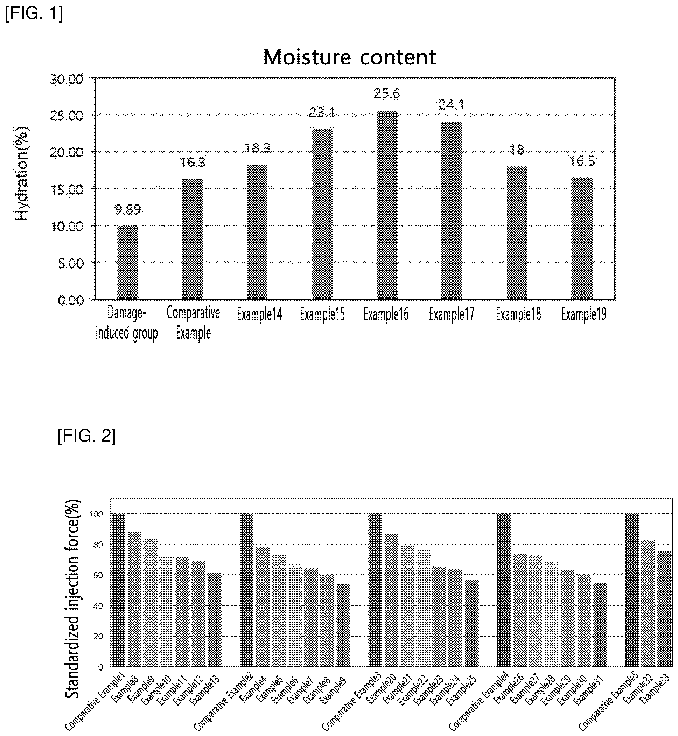

As a result, as shown in FIG. 1, it was confirmed that Examples comprising a specific content or ratio of glycerin to the DNA fragment mixture imparted higher hydration (moisturizing effect) to the tissue.

In particular, it was confirmed that the composition comprising the DNA fragment mixture in an amount of 2% by weight to 5% by weight with respect to the total weight of the composition and the polyhydric alcohol in an amount of 0.5% by weight to 4% by weight with respect to the total weight of the composition had critical significance for the moisturizing effect, and as described below, it was also confirmed that the composition had excellent tissue repair effects in terms of viscosity, injection force, injection feeling, biodegradability, etc. in the same content range, suggesting that the tissue repair effects with a higher engraftment rate and better moisturization may be obtained in the range of the aforementioned content combination.

### Experimental Example 2: Viscosity Test

The liquid compositions prepared in Comparative Examples 1 to 6 and Examples 8 to 33 with regard to the DNA fragment mixture alone, glycerin alone, and combinations of the DNA fragment mixture and glycerin were measured for complex viscosity (η*, Pa s; hereinafter, referred to as viscosity) using a rheometer (NETZSCH (Germany), Kinexus Ultra+ Rheometer) 48 hours after preparation. Specifically, the viscosity was measured under the following conditions: measurement temperature: 25°C, geometry used: PU20, gap: 1.0 mm, frequency: 0.1 Hz, shear strain: 0.5%, and analysis program: rSpace for Kinexus. The results are shown in Table 3 below.

**[Table 3]**

| Name of sample | Viscosity (η*, Pa·s) |
|---|---|
| Comparative Example 1 | 195.2 |
| Comparative Example 2 | 482.0 |
| Comparative Example 3 | 777.9 |
| Comparative Example 4 | 985.4 |
| Comparative Example 5 | 1924.0 |
| Comparative Example 6 | 0.03 |
| Example 8 | 122.2 |
| Example 9 | 43.12 |
| Example 10 | 8.176 |
| Example 11 | 5.697 |
| Example 12 | 3.266 |
| Example 13 | 2.651 |
| Example 14 | 276.1 |
| Example15 | 88.98 |
| Example 16 | 40.08 |
| Example 17 | 38.46 |
| Example 18 | 35.21 |
| Example 19 | 28.83 |
| Example 20 | 456.5 |
| Example 21 | 312.6 |
| Example 22 | 185.5 |
| Example 23 | 160.6 |
| Example 24 | 147.1 |
| Example 25 | 125.9 |
| Example 26 | 636.7 |
| Example 27 | 464.0 |
| Example 28 | 361.6 |
| Example 29 | 329.3 |
| Example 30 | 276.7 |
| Example 31 | 227.1 |
| Example 32 | 1237.0 |
| Example 33 | 1147.0 |

As a result, as shown in Table 3, it was confirmed that glycerin alone (Comparative Example 6) had a viscosity similar to purified water, and therefore, may not be used for tissue repair, whereas the viscosity of the combination of the DNA fragment mixture and glycerin (Examples 8 to 33) decreased, in comparison to the DNA fragment mixture alone (Comparative Examples 1 to 5). This suggests that the ease of injection of the combination of the DNA fragment mixture and glycerin (Examples 8 to 33) may increase.

### Experimental Example 3: Injection Force Test

Injection force was measured for Comparative Examples 1 to 5 and Examples 8 to 33, except for glycerin alone (Comparative Example 6) which was verified as not usable for tissue repair in Experimental Example 2.

The injection force represents a force (N) required to inject an injectable solution, and was measured at an injection speed of 50 mm/min. The liquid compositions prepared in all Examples and Comparative Examples for which injection force was examined were measured using an injection force tester (tensile and compression tester) 48 hours after preparation. Specifically, the injection force was measured using a tensile and compression tester (Universal Testing Machine (UTM), Daehwa Testing Machine, Republic of Korea) under the following conditions: measurement temperature of 25 ± 2°C, injection needle: JBP Korea (Republic of Korea), 33 G nano needle, and injection speed: 50 mm/min. The results are shown in Table 4 below and FIG. 2.

**[Table 4]**

| Name of sample | Injection force (N) |
|---|---|
| Comparative Example 1 | 17.2 |
| Comparative Example 2 | 27.17 |
| Comparative Example 3 | 32.37 |
| Comparative Example 4 | 49.33 |
| Comparative Example 5 | 69.39 |
| Example 8 | 15.22 |
| Example 9 | 14.45 |
| Example 10 | 12.47 |
| Example 11 | 12.34 |
| Example 12 | 11.88 |
| Example 13 | 10.49 |
| Example 14 | 21.3 |
| Example 15 | 19.81 |
| Example 16 | 18.15 |
| Example 17 | 17.4 |
| Example 18 | 16.21 |
| Example 19 | 14.66 |
| Example 20 | 28.09 |
| Example 21 | 25.69 |
| Example 22 | 24.79 |
| Example 23 | 21.3 |
| Example 24 | 20.68 |
| Example 25 | 18.28 |
| Example 26 | 36.46 |
| Example 27 | 35.87 |
| Example 28 | 33.66 |
| Example 29 | 31.13 |
| Example 30 | 29.4 |
| Example 31 | 26.88 |
| Example 32 | 52.43 |
| Example 33 | 48.05 |

The injection force values of the compositions without glycerin (Comparative Examples 1 to 5) were replaced with 100%, and based on the same content of DNA fragment mixture, the values of Examples were standardized to the values of Comparative Examples, and the results are shown as % values in FIG. 2.

As a result, as shown in Table 4 and FIG. 2, it was confirmed that the injection force was weakened in the case of the combination with glycerin, as compared to that of the DNA fragment mixture alone, and it was confirmed that even though Comparative Examples had higher injection forces, Examples with a combination of the DNA fragment mixture and glycerin had higher injection force control effects.

### Experimental Example 4: Injection Feeling Test

Excluding glycerin alone (Comparative Example 6) which was confirmed not to be useful for tissue repair in Experimental Example 2, and also excluding Examples 32 and 33 which did not satisfy that the injection force should be set to 40 N or less according to the guideline for approval of fillers for plastic surgery, representative Comparative Examples and Examples were evaluated for injection feeling.

A blind test was conducted with experimenters independently recruited (4 people), and the experimenters evaluated the injection feeling by releasing each composition of Comparative Examples and Experimental Examples into a petri dish using a syringe needle (33 gauge nano needle) used in actual products. Except for the use of a device (equipment) for measuring injection force, all other conditions were identical to those in the injection force test. 1 point was given for easier injection (more comfortable), and 5 points for more difficult injection (more uncomfortable). The results are shown in Tables 5 and 6 below. Table 5 shows the number of people who scored each score.

**[Table 5]**

| **Score** | Comparative Example 1 | Example 8 | Example 10 | Example 12 | Comparative Example 2 | Example 14 |
|---|---|---|---|---|---|---|
| **1** | | 2 | 3 | 4 | | |
| **2** | 3 | 2 | 1 | | | 1 |
| **3** | 1 | | | | 2 | 2 |
| **4** | | | | | 2 | 1 |
| **5** | | | | | | |
| **Total score** | 9 | 6 | 5 | 4 | 14 | 12 |
| **Injection force (N)** | 17.2 | 15.22 | 12.47 | 11.88 | 27.17 | 21.3 |

**[Table 6]**

| **Score** | Example 16 | Example 18 | Comparative Example 4 | Example 26 | Example 28 | Example 30 |
|---|---|---|---|---|---|---|
| **1** | | 1 | | | | |
| **2** | 3 | 3 | | | | |
| **3** | 1 | | | | 1 | 2 |
| **4** | | | | 2 | 3 | 2 |
| **5** | | | 4 | 2 | | |
| **Total score** | 9 | 7 | 20 | 18 | 15 | 14 |
| **Injection force (N)** | 18.15 | 16.21 | 49.33 | 36.46 | 33.66 | 29.4 |

As shown in Tables 5 and 6, the actual injection feeling test score was lower in the case of the combination with glycerin (Example 8, etc.), indicating a superior injection feeling, compared to the DNA fragment mixture alone (Comparative Examples 1, 2, and 4).

### Experimental Example 5: Phase Angle Test

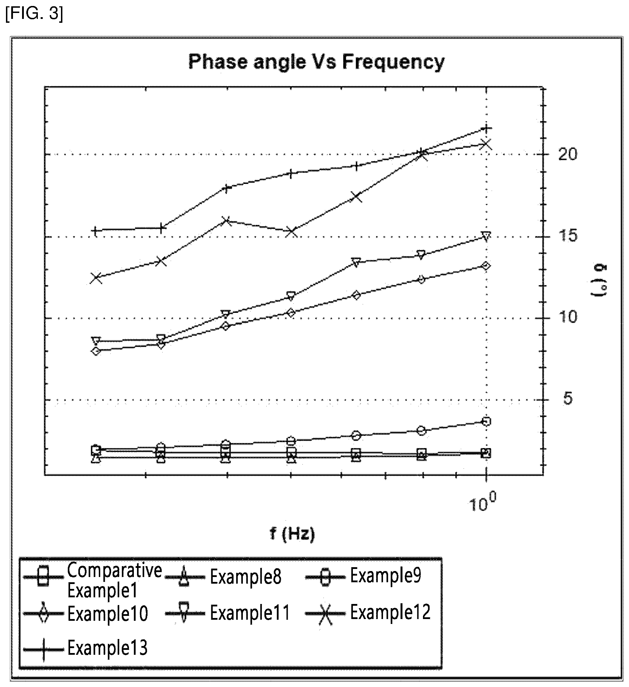

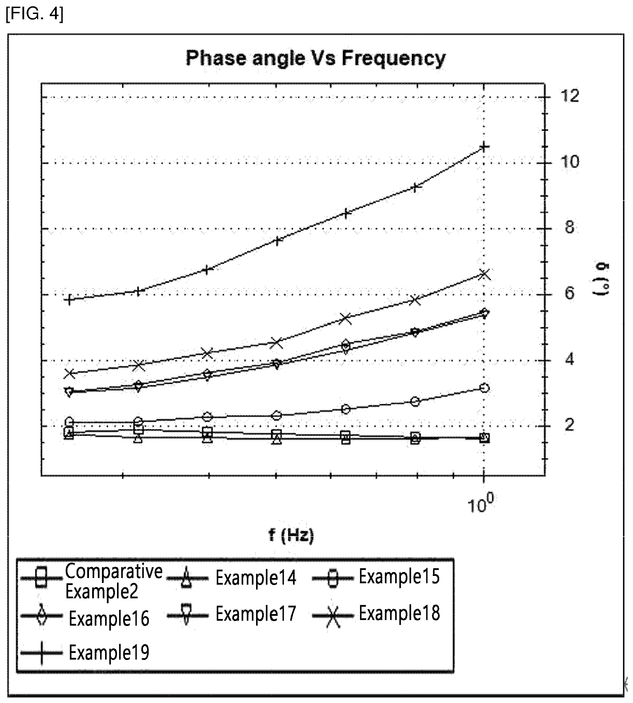

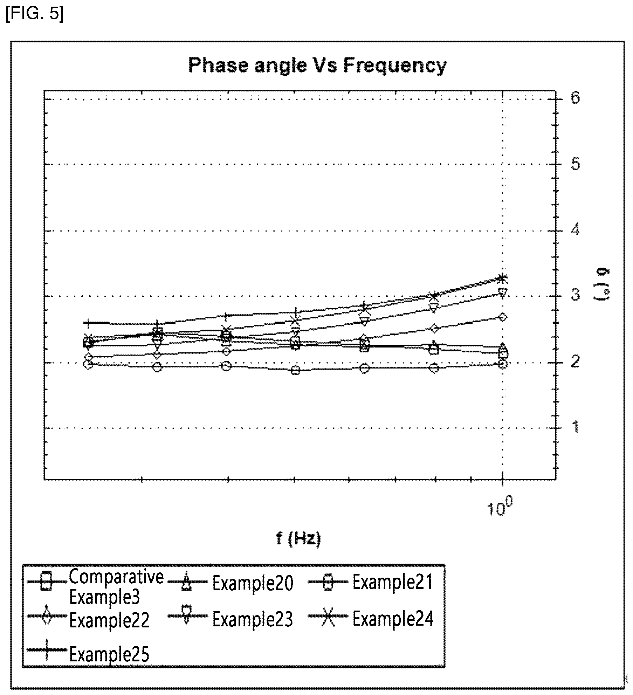

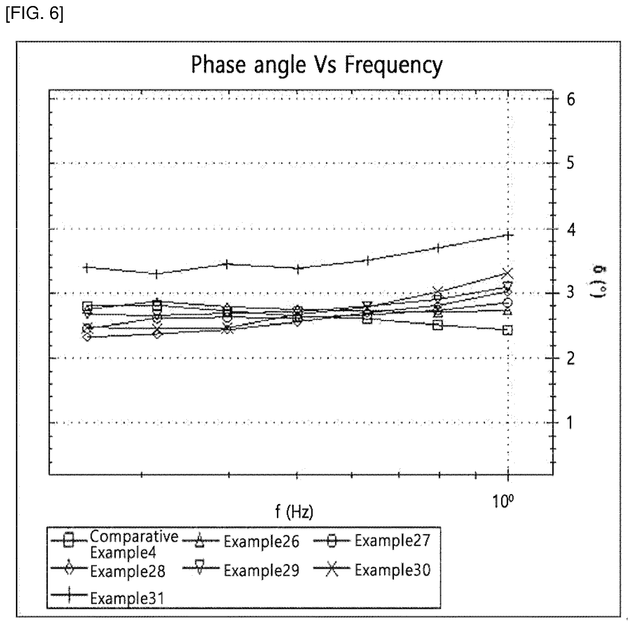

Each of the liquid compositions prepared in Examples and Comparative Examples was measured for phase angle (δ, °) using a rheometer (NETZSCH (Germany), Kinexus Ultra+ Rheometer) 48 hours after preparation. Specifically, the phase angle was measured under the following conditions: measurement temperature of 25°C, geometry used: PU20, gap: 1.0 mm, frequency: 0.1 Hz, shear strain: 0.5%, and analysis program: rSpace for Kinexus. and the results are shown in FIGS. 3 to 6.

As a result, as shown in FIGS. 3 to 6, the phase angle increased in the case of the combination with glycerin (Examples 8 to 31), as compared to the DNA fragment mixture alone (Comparative Examples 1 and 4), suggesting that the ease of injection may be increased by further increasing the fluidity.

### Experimental Example 6: Comparison of Biodegradability of Biomaterials for Repair according to Presence or Absence of DNA fragment mixture and Polyhydric alcohol

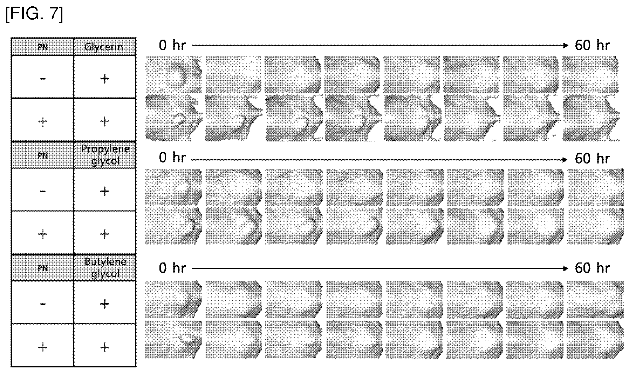

The liquid compositions, each comprising the DNA fragment mixture and/or polyhydric alcohol (glycerin, propylene glycol, butylene glycol) in the range of Comparative Example 6 and Example 28, were injected into a mouse body, respectively. The mice used were SKH-1 hairless mice (6 weeks old, female), and bred under the following conditions: temperature of 22 ± 2°C, relative humidity of 50 ± 10%, with food and water available *ad libitum.* The experimental period consisted of a 1-week acclimation period, followed by sample injection, and euthanasia 60 hours post-injection. A volume of 100 µL was injected intradermally into the dorsal region of the mice. Additionally, the volume change at the injection site after the sample injection was observed. The results of examining the same using 3D photo simulation (Primos, Canfield (USA)) are shown in FIG. 7.

As a result, as shown in FIG. 7, it was confirmed that polyhydric alcohol (glycerin, propylene glycol, butylene glycol) alone was completely decomposed immediately after injection, and may not be used for tissue repair purposes. However, it was confirmed that the combinations of DNA fragment mixture and polyhydric alcohol (glycerin, propylene glycol, butylene glycol) existed even after 60 hours, indicating that they are suitable as biomaterials for repair.

### Experimental Example 7: Comparison of Height and Width After Injection of Liquid Composition

When the biomaterial for tissue repair spreads too widely after injected into the body, the repair effect may be minimal, and when the width is narrow and the height is high, it may be too prominent visually and aesthetically, causing problems such as bumps or hives. Therefore, the width and height after injection were evaluated.

In this experiment, SKH-1 hairless mice (6 weeks old, female) were used. The mice were bred under the following conditions: temperature of 22 ± 2°C, relative humidity of 50 ± 10%, with food and water available *ad libitum.* The experimental period consisted of a 1-week acclimation period, followed by sample injection, and measurements were taken immediately post-injection. The mice were euthanized after 7 days. A volume of 100 µL was injected intradermally into the dorsal region of the mice.

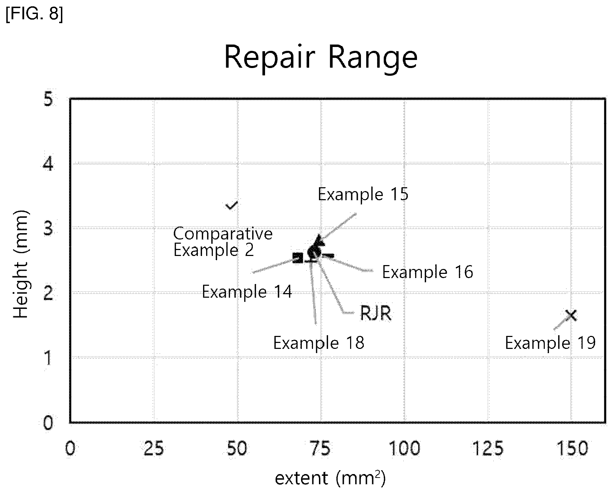

To this end, each of the liquid compositions of Comparative Examples and Examples prepared in Preparation Examples was injected into the body of a mouse, and the height and width where the sample was injected were measured using vernier calipers (MITUTOYO, Japan) and analyzed using ImageJ software, and the results are shown in FIG. 8 and Table 7.

**[Table 7]**

| | Width (mm²) | Height (mm) |
|---|---|---|
| RJR | 73.02 | 2.63 |
| Comparative Example 2 | 48.25 | 3.35 |
| Example 14 | 68.37 | 2.53 |
| Example 15 | 74.70 | 2.81 |
| Example 16 | 75.54 | 2.56 |
| Example 18 | 72.02 | 2.47 |
| Example 19 | 149.77 | 1.65 |

As shown in Table 7 and FIG. 8, compared to a commercial product (Rejuran; RJR; PharmaResearch Co., Ltd.), it was confirmed that the tested Examples 14, 15, 16 and 18 had a similar level of repair range to the commercial product, indicating that the compositions of the present disclosure have an excellent tissue repair effect.

### Experimental Example 8: Efficacy Comparison with Other Polyhydric Alcohols

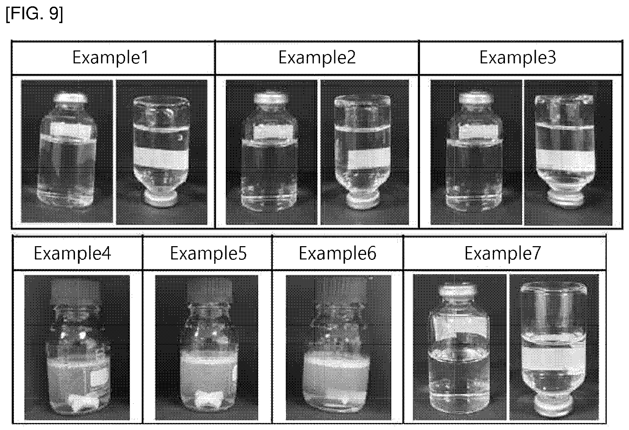

In order to compare the efficacy between Example 1 prepared using glycerin and Examples 2 to 7 prepared using other polyhydric alcohols instead of glycerin, their properties were compared and shown in FIG. 9.

As a result, as shown in FIG. 9, Examples 4 to 6 using other polyhydric alcohols generated a large amount of foam during the preparation of the liquid compositions, which may increase the probability of defective products during future product production, and thus it was confirmed that they may not be used as materials for tissue repair.

In addition, the results of evaluating the complex viscosity in the same manner as Experimental Example 2 for Examples 1 to 3, Example 7, and purified water, which did not generate foam, are shown in Table 8 below.

**[Table 8]**

| **Test item** | **Example 1** | **Example 2** | **Example 3** | **Example 7** | **Purified water** |
|---|---|---|---|---|---|
| **Viscosity (η*, Pa.s)** | 8.176 | 10.17 | 11.87 | 0.03771 | 0.02692 |

As a result, as shown in Table 8, it was confirmed that Example 7 may not be used as a biomaterial for tissue repair because its viscosity was as low as nearly the same as the level of purified water.

According to the results described above, it was confirmed that the composition for tissue repair comprising the DNA fragment mixture and polyhydric alcohol of the present disclosure not only has excellent tissue repair effect, but also has moisturizing effect in a specific content range combination. These results suggest that the composition does not excessively absorb moisture in the surrounding tissue when injected, but provides moisture for the surrounding tissue, and thus it does not cause dryness in the surrounding tissue but rather alleviates the dryness, and has a high engraftment rate and a good moisturizing effect.

## Claims

1. A composition for tissue repair, the composition comprising
a DNA fragment mixture; and a C3 or C4 polyhydric alcohol,
wherein the DNA fragment mixture is comprised in an amount of 2% by weight to 5% by weight with respect to the total weight of the composition,
the polyhydric alcohol is comprised in an amount of 0.5% by weight to 4% by weight with respect to the total weight of the composition, and
the polyhydric alcohol is any one or more selected from the group consisting of glycerin, propylene glycol, and butylene glycol.

2. The composition for tissue repair of claim 1, wherein the composition has a tissue moisturizing ability.

3. The composition for tissue repair of claim 1, wherein the composition increases a moisture content of a tissue when injected into the tissue.

4. The composition for tissue repair of claim 1, wherein the DNA fragment mixture and the polyhydric alcohol are comprised at a weight ratio of 10:1 to 1:2.

5. The composition for tissue repair of claim 1, wherein the DNA fragment mixture and the polyhydric alcohol are comprised at a weight ratio of 6:1 to 1:1.

6. The composition for tissue repair of claim 1, wherein the DNA fragment mixture is polynucleotide (PN), polydeoxyribonucleotide (PDRN), or a mixture thereof.

7. The composition for tissue repair of claim 1, wherein the DNA fragment mixture has a molecular weight of 50 kDa to 10,000 kDa.

8. A biomaterial for tissue repair, the biomaterial comprising the composition of any one of claims 1 to 7.

9. A filler composition comprising a DNA fragment mixture; and a C3 or C4 polyhydric alcohol,
wherein the DNA fragment mixture is comprised in an amount of 2% by weight to 5% by weight with respect to the total weight of the composition,
the polyhydric alcohol is comprised in an amount of 0.5% by weight to 4% by weight with respect to the total weight of the composition, and
the polyhydric alcohol is any one or more selected from the group consisting of glycerin, propylene glycol, and butylene glycol.

## Patentansprüche

1. Zusammensetzung für Gewebereparatur, die Zusammensetzung umfassend
ein DNA-Fragmentgemisch; und einen polyhydrischen C3- oder C4-Alkohol,
wobei das DNA-Fragmentgemisch in einer Menge von 2 Gew.-% bis 5 Gew.-% bezogen auf das Gesamtgewicht der Zusammensetzung enthalten ist,
der polyhydrische Alkohol in einer Menge von 0,5 Gew.-% bis 4 Gew.-% bezogen auf das Gesamtgewicht der Zusammensetzung enthalten ist, und
der polyhydrische Alkohol ein oder mehrere ist, die ausgewählt sind aus der Gruppe, bestehend aus Glycerin, Propylenglykol und Butylenglykol.

2. Zusammensetzung für Gewebereparatur nach Anspruch 1, wobei die Zusammensetzung eine Gewebebefeuchtungsfähigkeit aufweist.

3. Zusammensetzung für Gewebereparatur nach Anspruch 1, wobei die Zusammensetzung einen Feuchtigkeitsgehalt eines Gewebes erhöht, wenn sie in das Gewebe injiziert wird.

4. Zusammensetzung für Gewebereparatur nach Anspruch 1, wobei das DNA-Fragmentgemisch und der polyhydrische Alkohol in einem Gewichtsverhältnis von 10:1 bis 1:2 enthalten sind.

5. Zusammensetzung für Gewebereparatur nach Anspruch 1, wobei das DNA-Fragmentgemisch und der polyhydrische Alkohol in einem Gewichtsverhältnis von 6:1 bis 1:1 enthalten sind.

6. Zusammensetzung für Gewebereparatur nach Anspruch 1, wobei das DNA-Fragmentgemisch Polynukleotid (PN), Polydeoxyribonukleotid (PDRN) oder ein Gemisch davon ist.

7. Zusammensetzung für Gewebereparatur nach Anspruch 1, wobei das DNA-Fragmentgemisch ein Molekulargewicht von 50 kDa bis 10.000 kDa aufweist.

8. Biomaterial für Gewebereparatur, das Biomaterial umfassend die Zusammensetzung nach einem der Ansprüche 1 bis 7.

9. Füllstoffzusammensetzung, umfassend ein DNA-Fragmentgemisch und einen polyhydrischen C3- oder C4-Alkohol,
wobei das DNA-Fragmentgemisch in einer Menge von 2 Gew.-% bis 5 Gew.-% bezogen auf das Gesamtgewicht der Zusammensetzung enthalten ist,
der polyhydrische Alkohol in einer Menge von 0,5 Gew.-% bis 4 Gew.-% bezogen auf das Gesamtgewicht der Zusammensetzung enthalten ist und
der polyhydrische Alkohol ein oder mehrere ist, die ausgewählt sind aus der Gruppe, bestehend aus Glycerin, Propylenglykol und Butylenglykol.

## Revendications

1. Composition pour la réparation tissulaire, la composition comprenant
un mélange de fragments d'ADN ; et un alcool polyhydrique en C3 ou C4,
dans lequel le mélange de fragments d'ADN est présent en une quantité de 2 % en poids à 5 % en poids par rapport au poids total de la composition,
l'alcool polyhydrique est présent en une quantité de 0,5 % en poids à 4 % en poids par rapport au poids total de la composition, et
l'alcool polyhydrique est un ou plusieurs quelconques choisis dans le groupe constitué de glycérol, propylèneglycol et butylèneglycol.

2. Composition pour la réparation tissulaire selon la revendication 1, dans laquelle la composition présente une capacité d'hydratation tissulaire.

3. Composition pour la réparation tissulaire selon la revendication 1, dans laquelle la composition augmente la teneur en humidité d'un tissu lorsqu'elle est injectée dans le tissu.

4. Composition pour la réparation tissulaire selon la revendication 1, dans laquelle le mélange de fragments d'ADN et l'alcool polyhydrique sont présents dans un rapport pondéral de 10:1 à 1:2.

5. Composition pour la réparation tissulaire selon la revendication 1, dans laquelle le mélange de fragments d'ADN et l'alcool polyhydrique sont présents dans un rapport pondéral de 6:1 à 1:1.

6. Composition pour la réparation tissulaire selon la revendication 1, dans laquelle le mélange de fragments d'ADN est un polynucléotide (PN), un polydésoxyribonucléotide (PDRN), ou un mélange de ceux-ci.

7. Composition pour la réparation tissulaire selon la revendication 1, dans laquelle le mélange de fragments d'ADN présente un poids moléculaire de 50 kDa à 10 000 kDa.

8. Biomatériau pour la réparation tissulaire, le biomatériau comprenant la composition selon l'une quelconque des revendications 1 à 7.

9. Composition de comblement comprenant un mélange de fragments d'ADN ; et un alcool polyhydrique en C3 ou C4,
dans lequel le mélange de fragments d'ADN est présent en une quantité de 2 % en poids à 5 % en poids par rapport au poids total de la composition,
l'alcool polyhydrique est présent en une quantité de 0,5 % en poids à 4 % en poids par rapport au poids total de la composition, et
l'alcool polyhydrique est un ou plusieurs quelconques choisis dans le groupe constitué de glycérol, propylèneglycol et butylèneglycol.
